# EUROPÄISCHE PATENTSCHRIFT

(11) **EP 3 995 061 B1**
(45) Veröffentlichungstag und Bekanntmachung des Hinweises auf die Patenterteilung: **07.06.2023**
(21) Anmeldenummer: 21202846.8
(22) Anmeldetag: 15.10.2021
(51) Int. Cl.: A47L 5/24, A47L 7/00, A47L 9/02

(54) **VERFAHREN UND VORRICHTUNG ZUR REINIGUNG UND DESINFIZIERUNG EINER TEXTILOBERFLÄCHE SOWIE DESINFEKTIONSBOX**
DISINFECTION BOX AND METHOD AND DEVICE FOR CLEANING AND DISINFECTING A TEXTILE SURFACE
PROCÉDÉ ET DISPOSITIF DE NETTOYAGE ET DE DÉSINFECTION D'UNE SURFACE TEXTILE, AINSI QUE BOÎTE DE DÉSINFECTION

(30) Priorität: 09.11.2020 DE 102020129466
(43) Veröffentlichungstag der Anmeldung: 11.05.2022
(73) Patentinhaber: MattPro GmbH, 17429 Benz OT Labömitz (DE)
(72) Erfinder: Grzeskowiak, Eberhard, 17406 Usedom (DE)
(74) Vertreter: Werner, André

(56) Entgegenhaltungen:
- EP-A1- 3 138 455
- WO-A1-2010/019869
- DE-U1-202012 012 510
- DE-U1-202013 004 438
- US-A1- 2012 246 863

## Beschreibung

Die Erfindung betrifft ein Reinigungs- und Desinfektionsverfahren, mittels dessen eine mit biologischen Allergenen kontaminierte Textiloberfläche mit Hilfe von UVC-Strahlung sowie eines Staubsaugers reinigbar ist. Weiterhin betrifft die Erfindung eine Desinfektionsbox sowie eine Reinigungsvorrichtung umfassend selbige Desinfektionsbox. Anwendbar ist die Erfindung insbesondere für die Reinigung von Matratzen, Polstermöbeln oder Teppichen.

Matratzen, ebenso wie Teppiche und Polstermöbel, sind in der Regel mit abgestorbenen Hautzellen und Staubmilben belastet. Die Milben produzieren im Laufe ihres Lebens etwa das 200-fache ihres Gewichtes an Exkrementen und verwandeln so eine Matratze in ein Terrain mit höchster Allergenkonzentration.

Aus dem Stand der Technik sind Desinfektionsanlagen bekannt, die mittels UV-Strahlung Bakterien oder Mikroorganismen abtöten. Insbesondere durch Bestrahlen mit UVC bei 254 nm werden Bakterien inaktiviert.

Ein Verfahren und eine Vorrichtung zum Reinigen von Matratzen, Polstermöbeln und anderen textilen Gegenständen ist in DE 41 39 199 A1 gezeigt. Die Schrift zeigt eine Art Staubsaugerbürste mit einer integrierten Schwingungserzeugungsvorrichtung, die die zu reinigende Oberfläche mechanischen Schwingungen aussetzt, sodass anhaftende Schmutzpartikel gelockert bzw. zerkleinert werden. Mit Hilfe einer Unterdruckerzeugungsvorrichtung werden die Verschmutzungen abgesaugt. Zusätzlich kann eine Entkeimung mit ultravioletter Strahlung im Wellenlängenbereich von 273 nm vorgesehen sein.

Die DE 195 19 494 C1 beschreibt ein Verfahren zur Reinigung und Beseitigung von Hausstaubmilben aus Matratzen, bei welchem zusätzlich zur Saugbehandlung ein toxisches Gas, nämlich Kohlendioxid, in die Matratze eingeleitet wird. Hierfür ist jedoch stets ein Behälter mit dem Gas mitzuführen, was bei einer routinemäßigen Reinigung von z. B. Betten in einem Hotel unangemessen aufwendig ist.

EP 3 138 455 A1 offenbart einen Staubsauger zu Entfernung von Milben, der einen Staubsaugerkörper und eine Milbenentfernungsvorrichtung umfasst. Die Milbenentfernungsvorrichtung ist - in Form eines Anbaugerätes - lösbar mit dem vorderen Abschnitt des Staubsaugerkörpers verbunden; sie weist u. a. eine Leiterplatte und eine mit dieser elektrisch verbundene UV-Sterilisationslampe auf. Die UV-Sterilisationslampe befindet sich an der Unterseite der Milbenentfernungsvorrichtung.

Im Falle von Bettenreinigungen in Hotelzimmern besteht zudem die Gefahr, dass die Reinigungskräfte aus Zeitmangel die Matratzen nur unzureichend reinigen bzw. desinfizieren.

Ein weiterer Nachteil der o.g. Reinigungsverfahren liegt in einer mangelnden Berücksichtigung unterschiedlicher Desinfektionsanforderungen, sodass z. B. große Matratzen in derselben Zeitspanne wie kleine Matratzen gereinigt werden.

Der Erfindung liegt die Aufgabe zugrunde, die oben genannten Nachteile zu vermeiden, wobei ein Verfahren und eine Vorrichtung zur Reinigung und Desinfizierung von Textiloberflächen bereitgestellt werden soll, die eine sowohl trockene als auch umweltschonende antibakterielle Reinigung unter Verzicht auf chemische Reinigungsmittel ermöglicht, wobei sichergestellt werden soll, dass eine an die jeweilige Textiloberflächengröße angepasste, effektive Desinfektion durchgeführt wird.

Die Aufgabe wird durch ein Verfahren zur Reinigung einer Textiloberfläche und Desinfizierung mittels UVC-Strahlung mit den Merkmalen nach Patentanspruch 1, eine Desinfektionsbox zur Desinfizierung einer Textiloberfläche mit den Merkmalen nach Patentanspruch 7 sowie eine Vorrichtung zur Reinigung einer Textiloberfläche und Desinfizierung mittels UVC-Strahlung gemäß Patentanspruch 10 gelöst. Zweckmäßige Ausgestaltungen der Erfindung finden sich in den Unteransprüchen.

Gemäß der Offenbarung wird ein Verfahren zur Reinigung einer Textiloberfläche und Desinfizierung mittels UVC-Strahlung unter Verwendung eines Handstaubsaugers mit an demselben angeordneter Desinfektionsbox bereitgestellt, das ein Erfassen von Abbildungen der Textiloberfläche, ein automatisiertes Bestimmen der erforderlichen Reinigungsdauer sowie ein Erfassen der tatsächlichen Reinigungsdauer umfasst.

In einem ersten Verfahrensschritt wird mittels einer Kamera, die Bestandteil einer Steuereinrichtung sein kann, eine Abbildung der gesamten zu reinigenden Textiloberfläche erfasst. Weiterhin wird eine Flächengröße der zu reinigenden Textiloberfläche festgestellt. Dies kann entweder automatisiert von der Steuereinrichtung, z. B. anhand vorzugebender Merkmale, oder durch manuelle Eingabe in die Steuereinrichtung durch eine die Reinigung durchführende Reinigungskraft erfolgen.

Basierend auf der festgestellten Flächengröße wird von der Steuereinrichtung eine minimal notwendige Behandlungsdauer der Bestrahlung der Textiloberfläche mit UVC-Strahlung zur vollständigen Desinfektion bestimmt.

Anschließend erfolgt ein Reinigen der Textiloberfläche mittels des Handstaubsaugers und gleichzeitiger Desinfizierung mittels UVC-Strahlung wenigstens für die zuvor bestimmte minimal notwendige Behandlungsdauer. Während der Behandlung wird in vorgegebenen Zeitintervallen jeweils eine Abbildung von einem Teilbereich der Textiloberfläche mittels einer zweiten Kamera, die in oder an der Desinfektionsbox angeordnet ist, erfasst und in einer Datenspeichereinrichtung der Desinfektionsbox gespeichert oder an eine Datenspeichereinrichtung der Steuereinrichtung übertragen. Diese Zeitintervalle können im Millisekundenbereich liegen, d. h., es wird ein Film erstellt, oder auch bis zu mehrere Minuten betragen, sodass nur Einzelbilder erfasst und gespeichert werden. Es kann auch vorgesehen sein, je Reinigungsvorgang nur zwei Abbildungen mit der zweiten Kamera zu erfassen.

Nach Ablauf der minimal notwendigen Behandlungsdauer wird der Reinigungskraft optisch und/oder akustisch, z. B. von der Steuereinrichtung, signalisiert, dass die Reinigung beendet werden kann.

Schließlich wird noch eine Abbildung der gesamten, nunmehr gereinigten Textiloberfläche mittels der ersten Kamera erfasst.

Diese beiden mittels der ersten Kamera erfassten Abbildungen der gesamten Textiloberfläche werden zusammen mit der zuvor bestimmten minimal notwendigen Behandlungsdauer und der tatsächlichen Reinigungsdauer sowie einer Bezeichnung, z. B. Name oder Identifikationsnummer, der die Reinigung durchführenden Person, z. B. in einem Datenspeicher der Steuereinrichtung, abgespeichert.

Die Steuereinrichtung kann ein handelsübliches Smartphone mit Kamera sein, wobei auf dem Smartphone eine dedizierte Reinigungssteuersoftware (eine sog. App) installiert ist.

Da die Reinigungsdauer anhand der Größe der zu reinigenden Textiloberfläche von der Steuereinrichtung bestimmt wird, ist stets eine ausreichende und effektive Desinfektion garantiert. Durch die permanente Bilderfassung mittels der zweiten Kamera kann auch im Nachhinein sichergestellt werden, dass die Reinigungskraft die Textiloberfläche tatsächlich während der gesamten vorbestimmten notwendigen Behandlungsdauer bearbeitete.

Somit ist in Verbindung mit den Daten zu Reinigungsdauer sowie den Abbildungen der Textiloberfläche vor und nach der Reinigung eine Qualitätssicherung ermöglicht.

Durch den Verzicht auf zusätzliche chemische Reinigungsmittel entstehen kein Geruch und keine Umweltbelastung. Auch werden aufgrund der UV-basierten Desinfektion chemisch-resistente Erreger abgetötet.

Das erfindungsgemäße Verfahren kann weiter derart ausgebildet sein, dass neben den oben genannten Daten zusätzlich der Standort der gereinigten Textiloberfläche anhand von GPS-Daten sowie das aktuelle Datum und die Uhrzeit erfasst und abgespeichert werden. Hierzu wird die Datenspeichereinrichtung der Desinfektionsbox, vorzugsweise jedoch die Datenspeichereinrichtung der Steuereinrichtung verwendet.

Ferner kann vorgesehen sein, alle gespeicherten Daten, d. h. die in der Datenspeichereinrichtung der Desinfektionsbox sowie in der Datenspeichereinrichtung der Steuereinrichtung gespeicherten Daten, mindestens alle 24 Stunden, z. B. am Ende eines Arbeitstages oder nach jeder durchgeführten Reinigung, drahtlos auf einen Server zu übertragen. Dies kann über eine Internet-Verbindung, z. B. mittels WLAN, erfolgen. Hierzu weist die Steuereinrichtung ein Kommunikationsmodul zur Verbindung an das Internet auf.

Der Server ist vorzugsweise mit dem Internet verbunden, wobei durch eine z. B. grafische Schnittstelle ein Zugriff über das Internet auf die gespeicherten Daten des Servers ermöglicht ist. So kann in vorteilhafter Weise von jedem beliebigen Ort, an dem eine Verbindung zum Internet verfügbar ist, die Reinigung nachgeprüft werden.

Gemäß einer Ausführungsform wird ein aufgrund Erreichen der maximalen Lebensdauer einer die UVC-Strahlung erzeugenden UVC-Lampe notwendiger Austausch der UVC-Lampe optisch und/oder akustisch signalisiert. Diese Signalisierung kann mittels einer an oder in der Desinfektionsbox angeordneten Signalisierungseinrichtung oder durch ein von der Steuereinrichtung erzeugtes Signal erfolgen. Somit ist sichergestellt, dass die zur Desinfektion eingesetzte UVC-Strahlung stets die notwendige Intensität aufweist.

Ggf. kann es zweckmäßig sein, die Textiloberfläche vor oder nach der Reinigung mit einem flüssigen oder gasförmigen Desinfektionsmittel zu behandeln.

Zusätzlich oder alternativ kann vorgesehen sein, dass die Textiloberfläche beispielsweise mit Trockeneis auf eine Temperatur von unter -78,5 °C abgekühlt und hierdurch sterilisiert wird. Das Trockeneis kann in Form von Kohlendioxidschnee, z. B. mittels einer Sprühlanze, auf die Textiloberfläche aufgebracht werden, sodass diese hierdurch bei etwa -79 °C "schockgefrostet" wird.

Die offenbarungsgemäße Desinfektionsbox zur Desinfizierung einer Textiloberfläche umfasst wenigstens eine, vorzugsweise länglich ausgebildete Lichtquelle zur Emission von UVC-Licht in Richtung der Textiloberfläche, ein Vorschaltgerät zur Regelung von Leistung und Lichtfrequenz der Lichtquelle, eine auf die Textiloberfläche gerichtete Kamera, eine mit derselben verbundene Datenspeichereinrichtung und eine mit der Datenspeichereinrichtung verbundene Drahtlosschnittstelle.

Ein Vorteil ist, dass aufgrund eines regelnden Vorschaltgerätes die zur Desinfektion eingesetzte UVC-Strahlung stets mit der benötigten Intensität und Wellenlänge auf die Textiloberfläche eingestrahlt wird. Intensitätsschwankungen während eines Reinigungsvorganges oder mit zunehmendem Alter der UVC-Lichtquelle sind somit ausgeschlossen, sodass die aufgabengemäße effektive Desinfektion sichergestellt ist.

Die Drahtlosschnittstelle kann ein Bluetooth-Modul oder ein WLAN-Modul sein. Mittels der Drahtlosschnittstelle sind die von der Desinfektionsbox erfassten Daten bzw. Bilder zu einer Steuereinrichtung und/oder über das Internet auf einen dedizierten Server transferierbar.

Zusätzlich kann vorgesehen sein, die Desinfektionsbox über die Drahtlosschnittstelle zu steuern, insbesondere die UVC-Lichtquelle ein- und auszuschalten.

Bevorzugt ist als UVC-Lichtquelle eine Gasentladungsröhre eingesetzt. Hierbei weist die Desinfektionsbox zusätzlich einen Splitterschutz auf, der im Falle eines Bruches der Gasentladungsröhre vor umherfliegenden Glassplittern schützt. Die UVC-Lichtquelle selbst ist mit einem speziellen Gehäuse ausgestattet, welches Metallreflektoren an seiner Innenseite aufweist. Somit wird durch Reflexion eine maximale, gerichtete UV-Emission erreicht.

Die UVC-Lichtquelle weist bevorzugt eine Leistung von wenigstens 27 Watt, vorzugsweise von wenigstens 42 Watt, insbesondere von wenigstens 59 Watt auf.

Eine Ausgestaltung sieht vor, dass die Desinfektionsbox zwei länglich ausgebildete UVC-Lichtquellen, insbesondere zwei Gasentladungsröhren, umfasst.

Weiter kann vorgesehen werden, dass die UVC-Lichtquelle im Betrieb UVC-Strahlung in einem Wellenlängenbereich von weniger als 275 nm, vorzugsweise von im Wesentlichen 253 nm, emittiert. Aufgrund dieser kurzwelligen, energiereichen UV-Strahlung ist eine sehr effiziente keimtötende Wirkung erreicht.

Weiterhin kann die Desinfektionsbox eine Anzeigeeinheit aufweisen, mittels derer beispielsweise ein notwendiger Austausch der UVC-Lichtquelle signalisierbar und/oder eine Reinigungsdauer anzeigbar ist.

Die offenbarungsgemäße Reinigungsvorrichtung umfasst einen handelsüblichen Handstaubsauger, an dessen Stirnseite eine Desinfektionsbox mit den oben beschriebenen Merkmalen angeordnet ist. Vorzugseise ist ein Industriestaubsauger eingesetzt, der im Vergleich zu einem Haushalts-Staubsauger eine höhere Saugleistung aufweist. Aufgrund ihrer geringen Abmessungen und des geringen Gewichts kann die Reinigungsvorrichtung in vorteilhafter Weise zur mobilen Reinigung von Matratzen, z. B. in Hotelzimmern, eingesetzt werden. Durch den Verzicht auf zusätzliche Gastanks mit keimtötendem Gas ist die Reinigungsvorrichtung sehr handlich und kann somit auch von einer zierlichen Reinigungskraft von Zimmer zu Zimmer getragen und eingesetzt werden.

Weiterhin kann die Reinigungsvorrichtung mit einem Flüssigkeitstank fluidisch verbundene Düsen umfassen, die bei bestimmungsgemäßer Benutzung der Reinigungsvorrichtung auf die Textiloberfläche ausgerichtet sind. Hierdurch kann die zu reinigende Textiloberfläche mit einer Flüssigkeit besprüht werden, die geeignet ist, Bakterien oder andere Mikroorganismen abzutöten.

Die Erfindung wird nachfolgend anhand eines Ausführungsbeispiels näher erläutert, wobei gleiche oder ähnliche Merkmale mit gleichen Bezugszeichen versehen sind. Dazu zeigen in schematischer Darstellung die
- Fig. 1: eine Ausgestaltung der Reinigungsvorrichtung zum Reinigen und Desinfizieren einer Textiloberfläche in Schrägdraufsicht; und
- Fig. 2: eine Ausgestaltung der Desinfektionsbox im Längsschnitt.

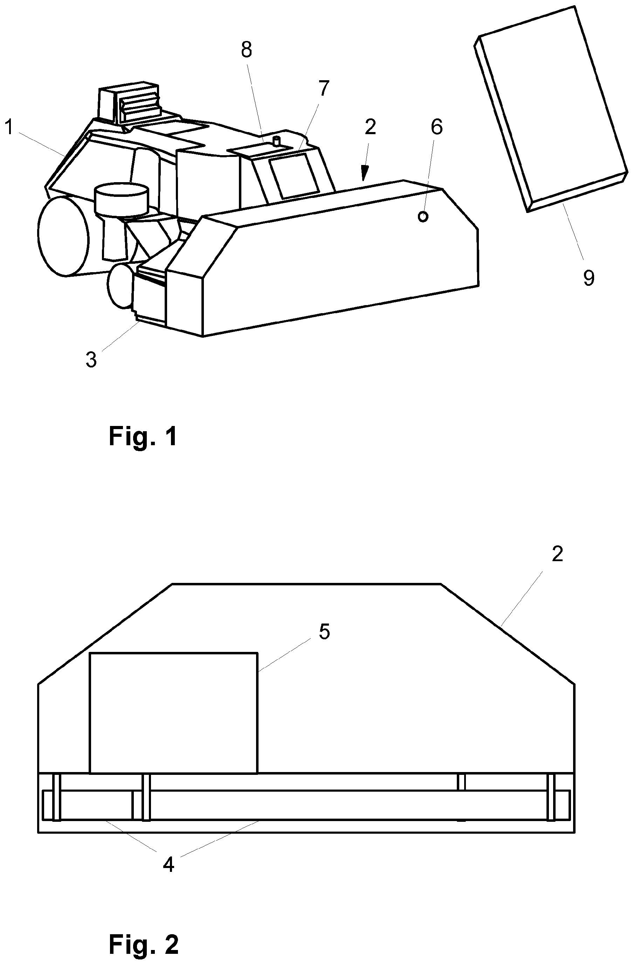

Die Reinigungsvorrichtung zum Reinigen und Desinfizieren einer Textiloberfläche gemäß Fig. 1 umfasst den Handstaubsauger 1, an dessen Stirnseite die Desinfektionsbox 2 derart angeordnet ist, dass sie - bzw. deren länglich ausgebildete UVC-Lichtquelle (nicht dargestellt) - parallel zur Saugdüse 3 des Handstaubsaugers 1 ausgerichtet ist, wobei die Breite des von der UVC-Lichtquelle bestrahlten Bereiches auf der Textiloberfläche im Wesentlichen der Breite des von der Saugdüse 3 erfassten Bereiches entspricht.

Die Desinfektionsbox 2 umfasst an ihrer Vorderseite die nach schräg unten gerichtete Kamera 6 und die Anzeigeeinheit 7 sowie auf ihrer Oberseite die Drahtlosschnittstelle 8, hier in Form eines Bluetooth-Moduls.

Als Steuereinrichtung ist das Smartphone 9 verwendet, auf welchem eine Software (App) zur Bestimmung und Anzeige der notwendigen Behandlungsdauer sowie zur Kommunikation mit der Drahtlosschnittstelle 8 und einem an das Internet angebundenen Server (nicht dargestellt) installiert ist. Das Smartphone 9 beinhaltet auch eine Kamera auf seiner Rückseite (nicht dargestellt).

Die Desinfektionsbox 2 zur Desinfizierung einer Textiloberfläche gemäß Fig. 2 umfasst eine erste stabförmige UVC-Lichtquelle 4 sowie eine zweite stabförmige UVC-Lichtquelle 4 (in der Figur hinter der ersten UVC-Lichtquelle), wobei die UVC-Lichtquellen 4 in diesem Beispiel Gasentladungsröhren sind, die von dem Vorschaltgerät 5 angesteuert werden, sodass sie UV-Licht der Wellenlänge 253 nm mit jeweils einer Leistung von 42 W abstrahlen.

### Liste der verwendeten Bezugszeichen

- 1: Handstaubsauger
- 2: Desinfektionsbox
- 3: Saugdüse
- 4: UVC-Lichtquelle
- 5: Vorschaltgerät
- 6: Kamera
- 7: Anzeigeeinheit
- 8: Drahtlosschnittstelle
- 9: Smartphone

## Patentansprüche

1. Verfahren zur Reinigung und Desinfizierung einer Textiloberfläche unter Verwendung eines Handstaubsaugers mit an demselben angeordneter, UVC-Strahlung erzeugender Desinfektionsbox (2), **gekennzeichnet dadurch, dass** das Verfahren die folgenden Schritte aufweist:
- Erfassen einer Abbildung der gesamten Textiloberfläche mittels einer ersten Kamera;
- Feststellen einer Flächengröße der zu reinigenden Textiloberfläche,
- Bestimmen einer minimal notwendigen Behandlungsdauer der Bestrahlung der Textiloberfläche mit UVC-Strahlung zur vollständigen Desinfektion,
- Reinigen der Textiloberfläche mittels des Handstaubsaugers (1) und Desinfizierung mittels UVC-Strahlung für die Behandlungsdauer,
- wobei in vorgegebenen Zeitintervallen jeweils eine Abbildung von einem Teilbereich der Textiloberfläche mittels einer zweiten Kamera (6), die in oder an der Desinfektionsbox (2) angeordnet ist, erfasst und in einer Datenspeichereinrichtung der Desinfektionsbox (2) gespeichert wird;
- Erfassen einer Abbildung der gesamten gereinigten Textiloberfläche mittels der ersten Kamera;
- Abspeichern von Dauer der Reinigung sowie von einer Bezeichnung der die Reinigung durchführenden Person.

2. Verfahren nach Patentanspruch 1, **dadurch gekennzeichnet, dass** vor oder nach der Reinigung der Standort mittels GPS sowie das aktuelle Datum und die Uhrzeit erfasst und gespeichert werden.

3. Verfahren nach einem der Patentansprüche 1 bis 2, **dadurch gekennzeichnet, dass** ein die erste Kamera enthaltendes Smartphone (9) zum Erfassen der Abbildungen der gesamten Textiloberfläche verwendet wird, wobei mittels einer auf dem Smartphone laufenden Software die notwendigen Behandlungsdauer der Bestrahlung bestimmt wird.

4. Verfahren nach einem der Patentansprüche 1 bis 3, **dadurch gekennzeichnet, dass** in der Datenspeichereinrichtung der Desinfektionsbox (2) gespeicherte Daten mindestens alle 24 Stunden drahtlos auf einen Server übertragen werden.

5. Verfahren nach einem der Patentansprüche 1 bis 4, **dadurch gekennzeichnet, dass** ein aufgrund Erreichen der maximalen Lebensdauer einer die UVC-Strahlung erzeugenden UVC-Lichtquelle (4) notwendiger Austausch der UVC-Lichtquelle (4) optisch und/oder akustisch signalisiert wird.

6. Verfahren nach einem der vorstehenden Patentansprüche, **dadurch gekennzeichnet, dass** die Textiloberfläche vor oder nach der Reinigung mit einem flüssigen oder gasförmigen Desinfektionsmittel behandelt wird.

7. Desinfektionsbox zur Desinfizierung einer Textiloberfläche, umfassend
- wenigstens eine, länglich ausgebildete UVC-Lichtquelle (4) zur Emission von UVC-Licht in Richtung der Textiloberfläche; **gekennzeichnet dadurch, dass** sie zusätzlich umfasst:
- ein Vorschaltgerät (5) zur Regelung von Lichtleistung und Lichtfrequenz des von der UVC-Lichtquelle (4) emittierten UVC-Lichts;
- eine auf die Textiloberfläche gerichtete Kamera (6);
- eine mit der Kamera (6) verbundene Datenspeichereinrichtung; und
- eine mit der Datenspeichereinrichtung verbundene Drahtlosschnittstelle (8).

8. Desinfektionsbox nach Anspruch 7, **dadurch gekennzeichnet, dass** die länglich ausgebildete UVC-Lichtquelle (4) zur Emission von UVC-Licht eine Gasentladungsröhre ist, wobei die Desinfektionsbox zusätzlich einen zumindest teilweise um die Gasentladungsröhre angeordneten Splitterschutz umfasst.

9. Desinfektionsbox nach Anspruch 7, **dadurch gekennzeichnet, dass** die UVC-Lichtquelle (4) ausgelegt ist, UVC-Licht mit einer Wellenlänge von im Wesentlichen 253 nm bei einer Leistung von wenigstens 27 Watt zu emittieren.

10. Reinigungsvorrichtung zur Reinigung und Desinfizierung einer Textiloberfläche, umfassend einen Handstaubsauger (1), an dessen Stirnseite eine Desinfektionsbox (2) nach Anspruch 7 angeordnet ist.

## Claims

1. Method for cleaning and disinfecting a textile surface using a hand-held vacuum cleaner with a disinfection box (2) arranged on it and generating UVC radiation, **characterized in that** method comprises the following steps:
- capturing an image of the entire textile surface by means of a first camera;
- determining a surface area size of the textile surface to be cleaned;
- determining a minimum required duration of irradiating the textile surface with UVC radiation for complete disinfection,
- cleaning of the textile surface using the hand vacuum cleaner (1) and disinfection using UVC radiation for the duration of the treatment,
- wherein at predetermined time intervals an image of a partial region of the textile surface is captured by means of a second camera (6) arranged in or on the disinfection box (2) and stored in a data storage device of the disinfection box (2);
- capturing an image of the entire cleaned textile surface by means of the first camera;
- saving a duration of the cleaning and a designation of the person performing the cleaning.

2. Method according to claim 1, **characterized in that** prior to or after the cleaning a location determined using GPS as well as the current date and time are saved.

3. Method according to claim 1 or 2, **characterized in that** a smartphone (9) comprising the first camera is used for capturing the image of the entire textile surface, wherein the minimum required duration of irradiating the textile surface is determined by use of a software application running on the smartphone (9)

4. Method according to one of claims 1 to 3, **characterized in that** data stored in the data storage device of the disinfection box (2) is transmitted wirelessly to a server at least every 24 hours.

5. Method according to one of claims 1 to 4, **characterized in that** a necessary replacement of the UVC light source (4) due to reaching the maximum service life of said UVC light source (4) generating the UVC radiation is signaled optically and/or acoustically.

6. Method according to one of the preceding patent claims, **characterized in that** the textile surface is treated using a liquid or gaseous disinfectant prior to or after the cleaning.

7. Disinfection box for disinfecting a textile surface, comprising
- at least one UVC light source (4) of elongated shape for emitting UVC light in the direction of the textile surface;
**characterized in** the it comprises additionally
- a ballast (5) for controlling the light power and light frequency of the UVC light emitted by the UVC light source (4);
- a camera (6) directed towards the textile surface;
- a data storage device connected to the camera (6); and
- a wireless interface (8) connected to the data storage device.

8. The disinfection box according to claim 7, **characterized in that** the UVC light source (4) of elongated shape for emitting UVC light is a gas discharge tube, wherein the disinfection box additionally comprises a splinter shield arranged at least partially around the gas discharge tube.

9. The disinfection box according to claim 7, **characterized in that** the UVC light source (4) is designed to emit UVC light having a wavelength of substantially 253 nm at a power of at least 27 watts.

10. A device for cleaning and disinfecting a textile surface comprising a hand-held vacuum cleaner (1), at the front face of which is arranged a disinfection box according to claim 7.

## Revendications

1. Procédé de nettoyage et de désinfection d'une surface textile en utilisant un aspirateur à main avec une boîte de désinfection (2) disposée sur celui-ci et produisant un rayonnement UVC, **caractérisé en ce que** le procédé présente les étapes suivantes :
- saisie d'une image de la totalité de la surface textile au moyen d'une première caméra ;
- détermination d'une taille de surface de la surface textile à nettoyer,
- détermination d'une durée de traitement minimale nécessaire à l'irradiation de la surface textile par rayonnement UVC pour une désinfection complète,
- nettoyage de la surface textile à l'aide de l'aspirateur à main (1) et désinfection par rayonnement UVC pendant la durée du traitement,
- une image d'une zone partielle de la surface textile étant respectivement saisie à des intervalles de temps prédéfinis au moyen d'une deuxième caméra (6), qui est disposée dans ou sur la boîte de désinfection (2), et étant enregistrée dans un dispositif de stockage de données de la boîte de désinfection (2) ;
- saisie d'une image de la totalité de la surface textile nettoyée au moyen de la première caméra ;
- mémorisation de la durée du nettoyage ainsi que d'une désignation de la personne effectuant le nettoyage.

2. Procédé selon la revendication 1, **caractérisé en ce qu'**avant ou après le nettoyage, l'emplacement au moyen du GPS ainsi que la date et l'heure actuelles sont saisis et enregistrés.

3. Procédé selon l'une des revendications 1 à 2, **caractérisé en ce qu'**un smartphone (9) contenant la première caméra est utilisé pour saisir les images de la totalité de la surface textile, la durée de traitement nécessaire de l'irradiation étant déterminée au moyen d'un logiciel fonctionnant sur le smartphone.

4. Procédé selon l'une des revendications 1 à 3, **caractérisé en ce que** des données enregistrées dans le dispositif de stockage de données de la boîte de désinfection (2) sont transmises sans fil à un serveur au moins toutes les 24 heures.

5. Procédé selon l'une des revendications 1 à 4, **caractérisé en ce qu'**un remplacement d'une source lumineuse UVC (4) nécessaire en raison de l'atteinte de la durée de vie maximale de la source lumineuse UVC (4) générant le rayonnement UVC est signalé de manière optique et/ou acoustique.

6. Procédé selon l'une des revendications précédentes, **caractérisé en ce que** la surface textile est traitée avant ou après le nettoyage avec un agent désinfectant liquide ou gazeux.

7. Boîte de désinfection pour la désinfection d'une surface textile, comprenant
- au moins une source lumineuse UVC (4) de forme allongée pour émettre de la lumière UVC en direction de la surface textile ; **caractérisée en ce qu'**elle comprend en outre :
- une résistance fixe (5) pour la régulation de la puissance lumineuse et de la fréquence lumineuse de la lumière UVC émise par la source lumineuse UVC (4) ;
- une caméra (6) dirigée vers la surface textile ;
- un dispositif de stockage de données connecté à la caméra (6) ; et
- une interface sans fil (8) connectée au dispositif de stockage de données.

8. Boîte de désinfection selon la revendication 7, **caractérisée en ce que** la source lumineuse UVC (4) de forme allongée pour émettre de la lumière UVC est un tube à décharge de gaz, la boîte de désinfection comprenant en outre une protection anti-éclats disposée au moins partiellement autour du tube à décharge de gaz.

9. Boîte de désinfection selon la revendication 7, **caractérisée en ce que** la source lumineuse UVC (4) est adaptée pour émettre de la lumière UVC ayant une longueur d'onde d'essentiellement 253 nm à une puissance d'au moins 27 watts.

10. Dispositif de nettoyage pour le nettoyage et la désinfection d'une surface textile, comprenant un aspirateur à main (1) sur la face frontale duquel est disposée une boîte de désinfection (2) selon la revendication 7.
